# EUROPEAN PATENT APPLICATION

(11) **EP 3 940 381 A1**
(43) Date of publication of application: **19.01.2022**
(21) Application number: 21185923.6
(22) Date of filing: 15.07.2021
(51) Int. Cl.: G01N 33/543

(54) **FUNCTIONALISED NANOPARTICLE**

(30) Priority: 17.07.2020 AU 2020902490
(71) Applicant: Radetec Pty Ltd, 3008 Melbourne, Victoria (AU)
(72) Inventor: Li, Zhiyuan, Brunswick, 3056 (AU); Lisi, Fabio, Brunswick, 3056 (AU)
(74) Representative: Barker Brettell LLP

(57) **Abstract**

A functionalised nanoparticle (10) that is at least in part coated by a polymer (14), wherein the polymer (14) comprises charged and uncharged groups at a ratio ranging from 4:1 to 1:4 and the functionalised nanoparticle (10) is conjugatable or can be functionalised to conjugate with a biomolecule (18).

## Description

### FIELD OF INVENTION

The present invention relates to a functionalised nanoparticle for detecting a target analyte. The functionalised nanoparticle may, for example but not exclusively, be conjugated with a biomolecule that can bond with the target analyte.

The present invention also relates to a method of producing such a functionalised nanoparticle.

### BACKGROUND

Nanoparticles have been used in biomedical research for a variety of applications including biosensors, drug delivery and imaging marking.

In recent years there has been an increasing demand for diagnostic devices for detecting a target analyte, such as pathogens, biomarkers, disease-specific antigens, pollutants and drugs.

Some devices, such as conventional lateral flow assay (LFA), use the conjugation chemistry between a label and a disease-generated analyte to detect the disease (e.g. gonorrhea and chlamydia). The most widely used label is colloidal gold. However, a large amount of gold-antibody conjugate is typically required for effective testing.

Consequently, one significant disadvantage of colloidal gold-based LFA is its inability to detect early stage infections which have a low pathogen load. This translates into poor clinical sensitivity and higher production cost.

Another challenge is that different reaction conditions are often required to conjugate each antibody to the gold particle which increases production cost.

It is beneficial to provide a material (e.g. nanoparticle) that can detect infections which have a low pathogen load. Suitably, the material can be modified to detect a wide range of infections.

### SUMMARY OF INVENTION

The present invention provides a functionalised nanoparticle that is at least in part coated by a polymer, wherein the polymer comprises charged and uncharged groups at a ratio ranging from 4:1 to 1:4 and the functionalised nanoparticle is conjugatable or can be functionalised to conjugate with a biomolecule.

The polymer may be coated on the nanoparticle in several ways. In one example, the polymer possesses a functional group that can react with a functional group on the nanoparticle to covalently bond the polymer to the nanoparticle. In another example, the polymer is bonded to the nanoparticle without reaction, for example by hydrogen bonding, ionic bonding or electrostatic forces.

The nanoparticle may have a diameter of less than 1 micron. Suitably, the nanoparticle has a diameter ranging from 1-100 nm. More suitably, the nanoparticle has a diameter ranging from 1-10 nm. Even more suitably, the nanoparticle has a diameter of about 9 nm,

The nanoparticle may comprise any one or more of polymeric, metallic, metal oxide or semi-conductor material.

The nanoparticle may comprise a quantum dot (QD). In this embodiment, the nanoparticle may be a composite including a QD. Suitably, the nanoparticle is a QD. More suitably, the QD is a core/shell QD.

The QD may comprise the following materials:
- Binary semiconductor group II-VI (e.g. CdSe, ZnS, CdS)
- Binary semiconductor group III-V (e.g. InP)
- Binary semiconductor group IV-VI (e.g. PbS)
- Ternary semiconductor group I-III-VI (e.g. CuInS₂)
- Quaternary semiconductor group I-II-IV-VI (e.g. Cu₂ZnSnS₄)

A preferred QD is a CdSe/ZnS core shell QD.

The charged and uncharged groups may be pendant functional groups.

The ratio of charged to uncharged groups may range from 3:1 to 1:3. It was discovered that increasing the ratio above 4:1 (i.e. more than 4 charged groups to uncharged groups) adversely impacts on the polymerisation process and reducing the ratio below 1:4 (i.e. less than 4 uncharged groups to charged groups) reduces solubility of the polymer in water.

Suitably, the ratio of charged to uncharged groups may be 1:1.

The applicant discovered that a ratio of 1:1 optimises solubility and stability of the nanoparticle.

The charged groups may have the same charge. Suitably, all the charged groups are negatively charged.

Alternatively, the charged groups in the polymer comprise a mixture of positively and negatively charged groups that provide a net neutral overall charge. The applicant discovered that an overall charge improves the stability of the particle in solution. In contrast, a net neutral charge may make the particle unstable.

At least one of the charged groups may be a sulfonate group. Suitably, all the charged groups are sulfonate groups. It was discovered that the sulfonate group improves solubility of the polymer in water.

At least one of the uncharged groups is a maleate group.

The uncharged groups may comprise a mixture of different uncharged groups.

The polymer may have a poly(styrene-maleic anhydride) backbone. The maleic anhydride functional group assists in post-polymerisation modification of the polymer.

The polymer may have a pendant functional group that can conjugate with a linker or a biomolecule.

The pendant functional group may be a thiol group or an azide group. Other suitable functional groups are carboxyl and hydroxyl groups.

The polymer may include pendant polyethyleneglycol (PEG) chains. The PEG chains may have a molecular weight (M_{w}) ranging from 100-5,000. Suitably, the PEG chains have a molecular weight (M_{w}) ranging from 600-900.

The applicant discovered that PEG can be used to reduce 'non-specific adsorption' (or 'non-specific binding') of unwanted proteins on the functionalised nanoparticle. PEG can also reduce adhesion of the coated particle on non-target substrates. This addresses one problem faced by existing diagnostic devices (e.g. if the particles randomly sticks on the nitrocellulose, instead of the test line), because such binding of nanoparticles increases the background signal, which in turns lowers the overall sensitivity of the diagnostic device.

The polymer may have the following structures, wherein n = 500-5,000 and m = 500-5,000:

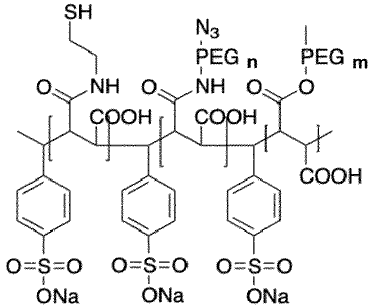

Suitably, n = 600-900 and m = 600-900

The functionalised nanoparticle may be conjugated to a biomolecule via a linker molecule. Suitably, the polymer on the functionalised nanoparticle may include a functional group that can bond to a functional group on the linker. The functional group on the functionalised nanoparticle may be an azide group and the functional group on the linker may be a cyclooctyne group.

The linker may react with the polymer to form a polymer-linker conjugate that can react with the nanoparticle to form the functionalised nanoparticle.

The functionalised nanoparticle may be conjugated to a biomolecule to form a nanoparticle-biomolecule conjugate that can bond, suitably complex, with a target analyte.

In order for the polymer to be conjugatable to a biomolecule, the polymer may have a functional group that can directly bond with the biomolecule or that can react with a linker that can bond with the biomolecule.

An example of a suitable linker is described in International patent application PCT/AU2013/000591.

The linker may comprise a cyclooctyne group and any one of more of a squarate, hydrazide, semicarbazide, carboxyl, maleimide, biotin, cyclopropene, norbonene, trans-cyclooctene, carbohydrazide, aminooxy or amine group.

The linker may be bonded to the polymer or biomolecule via a thiol-yne reaction.

The present invention also provides a method of producing a functionalised nanoparticle as previously described.

In one aspect, the present invention provides a method comprising:
providing a nanoparticle;
providing a polymer comprising charged and uncharged groups at a ratio ranging from 4:1 to 1:4; and
mixing the nanoparticle and the polymer to form the functionalised nanoparticle that is at least in part coated by the polymer and that is conjugatable or can be functionalised to conjugate to a biomolecule.

The method may include synthesizing a nanoparticle having a functional group that can react with a functional group on the polymer. Alternatively, the method may include modifying a nanoparticle to have a functional group that can react with a functional group on the polymer.

The method may include synthesizing a QD having a functional group that can react with a functional group on the polymer. Alternatively, the method may include modifying a commercially obtained QD with a functional group that can react with a functional group on the polymer.

The functional group may be any one of thiol, azide, carboxyl, hydroxyl and alkyne groups.

The nanoparticle and biomolecule may be bonded by "click chemistry".

The nanoparticle and biomolecule may be bonded by thiol-yne or azide-alkyne reactions. Examples of azide-alkyne reactions include metal-catalysed and strain-promoted cycloaddition.

The method may include synthesising the polymer comprising charged and uncharged groups at a ratio ranging from 4:1 to 1:4, suitably a ratio ranging from 3:1 to 1:3, more suitably a ratio of 1:1. The method may include functionalising the nanoparticle to enable polymerisation of monomers onto the nanoparticle to form the polymer. Suitably, the method includes polymerising the functionalised nanoparticle to form the polymer. In this embodiment, the polymer is synthesised on the nanoparticle.

The method may include reacting the nanoparticle with the polymer to covalently bond the nanoparticle and the polymer. The method of reacting the nanoparticle with the polymer may be performed at rtp (room temperature and pressure).

The method may include mixing the nanoparticle with the polymer to non-covalently bond the polymer to the nanoparticle, for example via electrostatic or hydrogen bonding.

The method may include functionalising the polymer to be conjugatable with a biomolecule. The step of functionalising the polymer may include functionalising the polymer to have one or more of thiol, azide, carboxyl, hydroxyl and alkyne pendant functional groups.

The method may include reacting the polymer with a linker to form a polymer-linker conjugate that is connectable to a biomolecule. Suitably, the polymer-linker conjugate possesses a pendant functional group that can react with a functional group on biomolecule to bond the biomolecule to the linker.

The present invention also provides a nanoparticle-biomolecule conjugate comprising a functionalised nanoparticle as previously described that is conjugated to a biomolecule, wherein the biomolecule can be bonded to a target analyte.

The biomolecule may be bonded to the target analyte in a number of ways including covalent bonding, non-covalent bonding including hydrogen bonding, ionic bonding, and complexation.

The analyte is detected by quantifying the fluorescent signal, that is proportional to the amount of analyte. The functionalized nanoparticle is such that if no analyte is present, no QDs will adhere to the substrate on which the analyte is adsorbed and thus no fluorescence would be detected.

The nanoparticle-biomolecule conjugate may fluoresce. Suitably, the fluorescence profile of the conjugate changes when the nanoparticle-biomolecule conjugate complexes with the target analyte. The change in the fluorescence profile may allow the target analyte to be detected.

The nanoparticle-biomolecule conjugate may comprise from 1 to 5 biomolecule(s). Suitably, the nanoparticle-biomolecule conjugate comprises from 1 to 3 biomolecule(s). More suitably, the nanoparticle-biomolecule conjugate comprises 1 biomolecule.

The biomolecule may be a polypeptide or a polynucleotide. The polypeptide may be a peptide or a protein. The polynucleotide may be an aptamer or a nucleic acid. Suitably, the biomolecule is an antibody. The antibody may be a polyclonal antibody or a monoclonal antibody. The antibody may be an anti-biotin antibody.

The target analyte may be an antigen, a nucleic acid or a biomarker. Suitably, the antigen, nucleic acid or biomarker is a disease-related molecule. The antigen, nucleic acid or biomarker may be located on the surface of a pathogen such as a bacteria or virus. The antigen, nucleic acid or biomarker may be found in biological fluids such as blood, saliva, vaginal fluid, etc. For example, the antibody on the nanoparticle-biomolecule conjugate may complex with a type of antigen, nucleic acid or biomarker. This may enable the conjugate to detect a general class of pathogens which can be used to diagnose a specific disease.

The present invention also provides a method of producing the previously described nanoparticle-biomolecule conjugate.

In one aspect, present invention also provides a method comprising:
providing a nanoparticle;
providing a polymer comprising charged and uncharged groups at a ratio ranging from 4:1 to 1:4;
mixing the nanoparticle and the polymer to form a functionalised nanoparticle which is at least in part coated by the polymer; and
reacting the functionalised nanoparticle with a biomolecule to form the nanoparticle-biomolecule conjugate, wherein the nanoparticle-biomolecule conjugate can be bonded to a target analyte.

The steps for producing the functionalised nanoparticle are as previously described.

The method may include reacting the polymer with a linker to form a polymer-linker conjugate that can bond to a biomolecule to form the nanoparticle-biomolecule conjugate. In this embodiment, the polymer-linker conjugate connects to the biomolecule non-covalently. The method may include reacting the polymer-linker conjugate with the biomolecule. In this embodiment, the polymer-linker conjugate connects to the biomolecule covalently.

The method may include reacting the polymer with the biomolecule to form the nanoparticle-biomolecule conjugate.

The method may include a step of reacting the linker with the biomolecule to form a biomolecule-linker conjugate.

This method may include reacting the biomolecule-linker conjugate with the polymer to form the nanoparticle-biomolecule conjugate.

The method may include purifying the nanoparticle-biomolecule conjugate by spin filtering.

The method may include storing the polymer-biomolecule conjugate in a Borate buffered saline (BBS) solution.

The method may include applying the polymer-biomolecule conjugate onto a substrate.

In this specification, the terms "bonding" and "conjugation" are used interchangeably.

In this specification, the term "biomolecule" refers to a molecule that is produced by a living organism.

In this specification, the term "linker" refers to a multi-functional molecule that can connect the coated nanoparticle to the biomolecule.

### BRIEF DESCRIPTION OF DRAWINGS

Figure 1 illustrates a nanoparticle-biomolecule conjugate according to an embodiment of the present invention.
Figure 2 illustrates the complexation of the nanoparticle-biomolecule conjugate of Figure 1 with an antigen.
Figure 3 is a flow diagram illustrating methods of producing a coated nanoparticle and a nanoparticle-biomolecule conjugate according to the present invention.

### DETAILED DESCRIPTION

The present invention relates to a nanoparticle-biomolecule conjugate that can be used to detect a disease, and a method of synthesising the conjugate.

One of the motivations of the present invention is the realization that fluorescence detection is considered to be more sensitive than colourimetric detectionbecause lower concentrations of fluorescent materials can be detected compared to coloured material. In some instances, it may be possible with the right instrument to detect a single QD or dye.

However, to achieve such sensitive detection, background signals should be minimized. The present invention achieves this by providing the polymeric coating, which minimises non-specific adsorption of the nanoparticles (e.g. QDs).

This enables the present invention to detect a disease with a combination of fluorescence detection and low non-specific adsorption.

QDs fluoresce brighter than other fluorescent molecules such as dyes.

Brightness is defined as the product of the molar absorption coefficient multiplied by fluorescence quantum yield.

Molar absorption coefficient for QDs ranges from 100,000-1,000,000 M⁻¹ cm⁻¹, while the coefficient for dyes ranges from 25,000-250,000 M⁻¹ cm⁻¹.

Quantum yield for dyes can range from 1% to over 90%, depending on the dye. Quantum yield for QDs can be up to 100%, but typically drops to 20% when the particles are dispersed into water.

One advantage of the present invention is that the quantum yield of the QD can be retained at about 80% in water.

Use of a QD in the functionalized nanoparticle may provide up to 4 times the brightness of dyes.

The high signal to analyte ratio allows detection of low concentrations of a target analyte such as a disease-specific antigen. As a result, the use of QDs as the nanoparticle can facilitate early diagnosis and treatment.

However, the use of QDs is not straightforward. One challenge faced by the applicant is the conjugation between QDs and antibodies, for example in their application with LFA, enzyme-linked immunosorbent assay (ELISA) or other methods. This is because different reaction conditions often have to be established for each antibody, resulting in increased costs. Often, these reaction conditions involve high temperatures and/or pressures, and extreme pH conditions.

The present invention provides a functionalized nanoparticle that can be conjugated with a range of antibodies using mild reaction conditions to form a nanoparticle-biomolecule conjugate.

Reaction temperatures may range from 4-30°C. Suitably, the reaction temperature ranges from 15-25°C.

Reaction pressures may range from 1-1.5 atm. Suitably, the reaction pressure is at 1 atm.

Reaction pH may range from 7 to 9. Suitably, the reaction pH ranges from 7 to 8. More suitably, the reaction pH ranges from 7 to 7.5.

The biomolecule concentration may range from 100 µM - 1 nM.

The nanoparticle-biomolecule conjugate comprises a nanoparticle that has an outer surface that is functionalized with a biomolecule, suitably an antibody, that can complex with a target analyte such as an antigen. However, it can be appreciated that the biomolecule can be any other type of protein.

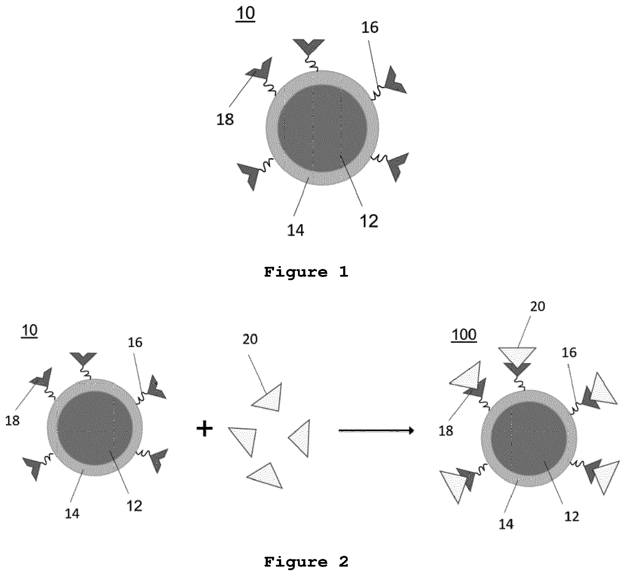

With reference to Figure 1, the nanoparticle-biomolecule conjugate 100 comprises a nanoparticle 12 in the form of a QD, suitably a CdSe/ZnS core shell QD.

It is believed that the QD is bonded to the polymer 14 via a combination of interaction between the -SH functional group on the polymer with the surface of the QD, and interaction between the aromatic ring and other portions of the polymer with the surface of the QD.

The polymer 14 that has a 1:1 ratio of negatively charged sulfonate groups (Group 4 molecules in the polymer examples below) and uncharged maleate groups to form a functionalised nanoparticle 10. Examples of suitable polymers (P1 and P2) are illustrated below:

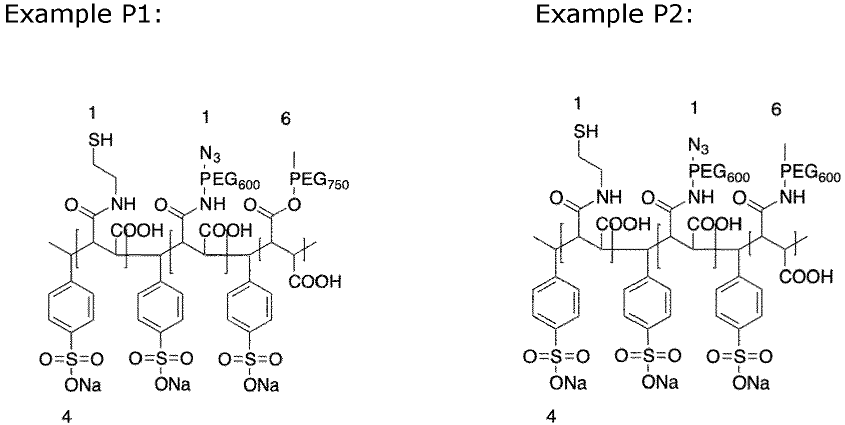

The polymer 14 has pendant thiol and azide groups (Group 1 molecules in the examples above). In some embodiments, the polymer may have pendant carboxyl and/or hydroxyl groups. These functional groups enable conjugation with a biomolecule.

Each polymer 14 also includes pendant polyethyleneglycol (PEG) chains having M_{w}s of 600 and 750 for P1 and 600 for P2 (Group 6 molecules in the examples).

The pendant azide group on the polymer-coated nanoparticle can react with alkyne groups on a di-functional linker 16 to form a functionalized nanoparticle that can conjugate with an antibody 18.

The synthesized polymer 1stabilizes the QD in water, provides the QD with a high quantum yield, low non-specific adsorption and allows the functionalised QD to be modified with a variety of functional groups for conjugation with a biomolecule.

The linker 16 may react with the polymer to form a polymer-linker conjugate that can react with an antibody 18 to form the nanoparticle-antibody conjugate 10.

Alternatively, the linker 16 is reacted with an antibody 18 to form a linker-antibody conjugate that can react with a polymer-coated nanoparticle to form the nanoparticle-antibody conjugate 10.

This provides an antibody-covered QD 10 can complex with a suitable antigen 20 which enables detection of a relevant pathogen (see Figure 2).

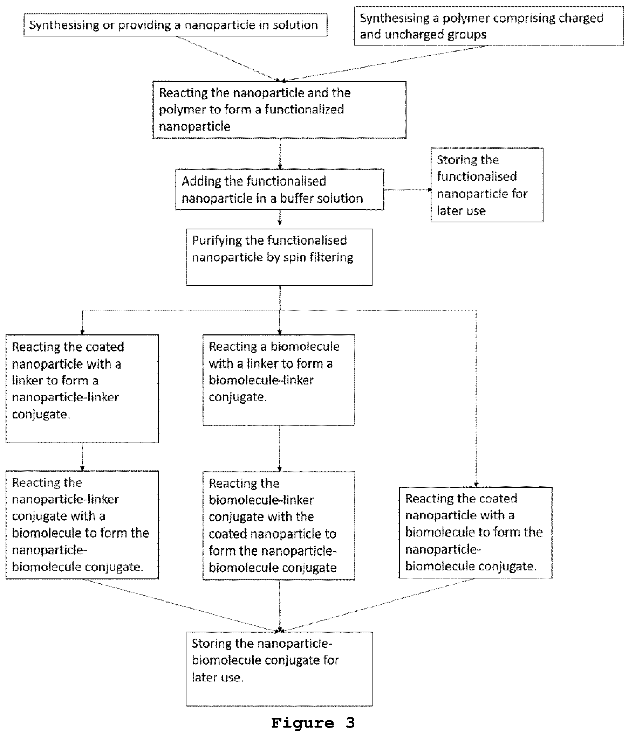

In the example illustrated in Figure 3, a nanoparticle-biomolecule conjugate 10 is formed by taking the following steps:
1. Separately synthesising/obtaining a nanoparticle 12 (e.g. a CdSe/ZnS core shell QD) and a polymer 14.
2. Reacting the nanoparticle 12 and polymer 14 to form a functionalised nanoparticle 10 in which the polymer 14 is bonded to the nanoparticle 12 and coats the outer surface of the nanoparticle 12.
3. The functionalised nanoparticle 10 is added to a buffer solution, preferably a BBS solution.
4. The functionalised nanoparticle 10 in solution can be stored for later use or processed to form the nanoparticle-biomolecule conjugate 100.
5. To form the nanoparticle-biomolecule conjugate 100, the functionalised nanoparticle 10 is purified by spin filtering. Thereafter, there are three synthetic pathways that can be taken to form the nanoparticle-biomolecule conjugate 100.
6. In pathway 1, the functionalised nanoparticle 10 is reacted with a linker 16 to form a nanoparticle-linker conjugate. The nanoparticle-linker conjugate is then reacted with a biomolecule 18, which in this example is an antibody, to form the nanoparticle-biomolecule conjugate 100.
7. In pathway 2, a biomolecule 18 such as an antibody is reacted with a linker 16 to form a biomolecule-linker conjugate. The biomolecule-linker conjugate is then reacted with the functionalised nanoparticle 10 to form the nanoparticle-biomolecule conjugate 100.
8. In pathway 3, the functionalised nanoparticle 10 is reacted directly with a biomolecule 18 to form the nanoparticle-biomolecule conjugate 100.
9. The nanoparticle-biomolecule conjugate 100 is stored in a buffer solution for future use.

In order to detect a target analyte, a solution of anti-ECM (MM01) antibody in PBS is spotted onto a test strip and dried.

A solution of ECM in QDs-anti-ECM-antibody in a washing buffer is then prepared. The ECM solution is applied to the antibody-loaded strip and dried. The dried strip is washed with wash buffer and dried again before being checked for fluorescence under UV light.

### EXAMPLE

### Polymer synthesis

### 1. α,ω-Dichloro-PEG600

Poly(ethylene glycol) (M_{W} 600, Sigma-Aldrich, 60.0 g) was dried by heating under vacuum for 1 h in an oil bath at 80-90°C with constant stirring, then cooled to ambient temperature. A solution of thionyl chloride (22 mL, 36 g, 300 mmol) in diethyl ether (38 mL) was added using a dropping funnel over 50 min. The mixture was stirred overnight at room temperature. The solvent was removed under reduced pressure with heating at 70°C, and the residue was diluted with CH₂Cl₂ (40 mL) and re-evaporated under reduced pressure with heating at 70°C . Nuclear magnetic resonance (NMR) was used to characterise the polymer with parameters as follows: 1H NMR δ 3.74-3.77 (4 H, t, CH₂Cl), 3.62-3.70 (45 H, m,CH₂O), 2.62 (0.24 H, m, OH). IR v (cm⁻¹) 2866 (C-H), 1097 (ether), 743 (C-Cl); no discernible -OH above 3100.

### 2. α,ω-bisazido-PEG600

Crude α,ω-dichloro-PEG600(41.5 g, 65 mmol) was dissolved in DMF (Dimethylformamide) (330 g). KHCO₃ (0.88 g) and NaN₃ (12.7 g, 196 mmol) were added. The mixture was stirred and heated at 80-90°C under nitrogen for 20 h. DMF was evaporated under vacuum and dichloromethane (275 mL) was added. Inorganic solids were removed by filtration and the solvent evaporated under reduced pressure at 50°C , yielding a yellow liquid (47.3 g, 87% pure as assessed by 1H NMR). Nuclear magnetic resonance (NMR) was used to characterise the polymer with parameters as follows :1H NMR δ 3.37-3.40 (4 H, t, CH₂N₃), 3.65-3.69 (49 H, m, CH₂O). Sample contains 1.3 mol of DMF per mol of bisazide (0.15 g/g).

### 3. α-Amino-ω-azido-PEG600(H2N-PEG-N3)

Crude α,ω-bisazido-PEG600 (47.2 g, 87% pure) was dissolved in 1 M HCl (184 mL) in a 1 L flask and cooled to 0°C. Toluene (380 mL) was added and the mixture stirred vigorously under a stream of nitrogen. A solution of triphenylphosphine (18.2 g, 69.6 mmol) in toluene (200 mL) was added using a dropping funnel over 2 h. The mixture was allowed to warm to room temperature and was stirred under nitrogen overnight. The aqueous layer was separated, washed with toluene (3 x 100 mL) and then chilled on ice. Solid KOH (90 g) was added to the aqueous phase, which was then extracted with CH₂Cl₂ (3 × 75 mL). The extract was dried (MgSO₄), filtered and the solvent evaporated, yielding 37 g of a light yellow oil. Nuclear magnetic resonance (NMR) was used to characterise the polymer with parameters as follows: 1H NMR δ 3.37-3.41 (2 H, t, CH₂N₃), 3.62- 3.69 (45 H, m, CH₂O), 3.52-3.55 (2 H, t, CH₂CH₂NH₂), 2.86-2.89 (2 H, t, CH₂NH₂), 2.17-2.21 (2 H, m, NH₂). IR v (cm-1) 3366 (NH₂, weak), 2100 (N₃), 1591 (amine, weak).

### 4. Polymer Backbone

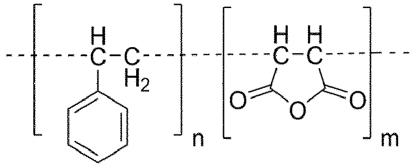

DoPAT (2-(Dodecylthiocarbonothioylthio)propionic acid) (0.35 g, 1 mmol) and AIBN (80 mg, 0.5 mmol) and maleic anhydride (3.675g, 37.5 mmol) was dissolved in 1,4 dioxane (10 mL). A second solution of styrenesulfonate (2.575 g, 12.5 mmol) in water (10 mL) was prepared. The two solutions were mixed together and degassed three times. The reaction was heated at 70°C for overnight. The solution was precipitated from acetone and centrifuged 3 times.

### 5. 10th generation (10^{th} G) polymer

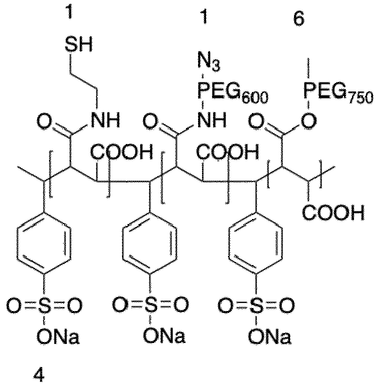

Polymer backbone of item 4 (180 mg, 0.92 mmol), cysteamine (17.9 mg, 0.148 mol (1/6)), CH₃-PEG-OH (Mw 750 g/mol, 666.1 mg, 0.888 mmol) and NH₂-PEG-N₃ (Mw 600 g/mol, 88.8 mg, 0.148 mmol) were added into water (3 mL). Another solution was prepared by dissolving EDC (1-Ethyl-3-[3-dimethylaminopropyl]carbodiimide hydrochloride, 568.4 mg, 2.96 mmol) and NHS (hydroxysuccinimide, 340.46 mg, 2.96 mmol) in water (10 mL). The above described two solutions were mixed together overnight. The resulting polymer was purified by spin filter three times (50K size, 6000 g, 10 mins).

### 6. 11^{th}-generation (11^{th} G) polymer

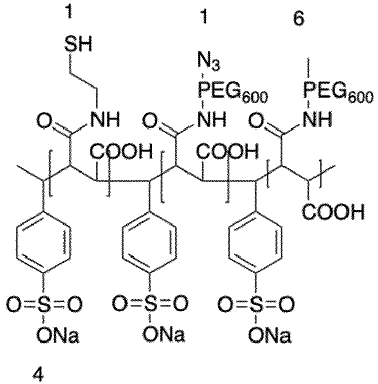

Polymer backbone of item 4 (180 mg, 0.92 mmol), cysteamine (17.9 mg, 0.148 mol (1/6)), *O*-(2-Aminopropyl)-*O'*-(2-methoxyethyl)polypropylene glycol (Mw 600 g/mol, 640.8 mg, 0.888 mmol) and NH₂-PEG-N₃ (Mw 600 g/mol, 88.8 mg, 0.148 mmol) were added into water (3 mL). Another solution was prepared by dissolving EDC (1-Ethyl-3-[3-dimethylaminopropyl]carbodiimide hydrochloride, 568.4 mg, 2.96 mmol) and NHS (hydroxysuccinimide, 340.46 mg, 2.96 mmol) in water (10 mL). Two solutions were mixed together overnight. The resulting polymer was purified by spin filter three times (50K size, 6000 g, 10 mins).

### QDs MUA (11-Mercaptoundecanoic acid) phase transfer

The phase transfer protocol described below was applied to different type of QDs solutions.
1. A QDs solution (in chloroform 3 mL) was precipitated with methanol, centrifuged and redispersed in hexane (9 mL).
2. A second solution was prepared by dissolving KOH (0.2 g) and MUA (2.78 mmol, 0.78 g) in methanol (9 mL).
3. Both solutions were mixed in a centrifuged tube and shaken overnight. (two ways, rotating vertically @ 30 rmp; or rotating horizontally @ 50 rmp).
4. After the phase transfer, the colourless hexane phase was separated from methanol phase.
5. To separate the QDs, the methanol phase was centrifuged. The precipitated QDs were redispersed in KOH solution (3 mL, 0.1 M). This solution was washed with chloroform.

### Ligand exchange with 10th G or 11th G polymer

1. A QDs solution (in MUA, 400 uL) was added into polymer solution (1 mL, 50 mg/mL).
2. The solution was mixed overnight (two ways, rotating vertically @30 rpm; or rotating horizontally @50 rpm).
3. The QDs solution was run a pre-packed desalting column to remove the MUA ligands. Using BBS buffer solution as the eluent.
4. The QDs solution in BBS buffer was further purified by spin filtering (three times, 6000 rcf, 10 min).

### Conjugation protocol

The conjugation protocol was carried out over two days as described below.

### DAY 1: Linker + antibody

1. Anti-biotin antibody (20uL, 2mg/ml, 0.04mg, 2.67e-10 mol) and linker (2*2.35 = 4.7e-10 mol, 0.1 mM, 4.71µL) in BBS buffer (40 uL) was mixed in a 200 µL tube for 18 hours at RT on an orbital shaker (750 rpm). For modification of thiol groups and -amino groups, which occurs selectively at physiological pH (7.0-7.5), phosphate buffers are ideally suited. More strongly basic lysine amines require more alkaline pH, in the range of 8.0-9.5, where phosphate solutions do not buffer well. For these reactions, carbonate/bicarbonate (pH of 100 mM bicarbonate is 9.2) or borate buffers are quite satisfactory. No additional buffer was added.
2. Quench with 50mM glycine.
3. Purify by spin filter (50K size) three times (6000 RCF, 8min) using BBS.
4. Collect the concentrated protein in a 200 µL tube, washing the filter once with 2-3 drops of BBS. The volume should be below ∼130 µL, to accommodate the QDs.
5. Characterise the modification via LC-ESI-TOF.

Note: The reaction can be refrigerated for 2 days instead of 18 hrs at RT.

### DAY 2: Linker-antibody conjugate + QDs

1. The antibody-linker (2.35e-10 mol) from the previous step was added to the QD (2.35 e-10 mol, 0.5 µM, 4.7e-4 L, 470 µL) in BBS buffer, and mixed for 18 hours at RT on an orbital shaker (750 rpm). The initial protocol used 5.88e-11 mol QDs (the ratio antibody:QD was 4:1).
2. Purify by spin filter (300K size) three times using PBS-T (*Invitrogen conditions:* 1500 RCF, 30 min; *quick:* 4000 RCF, 5 min). Gently resuspend the QD after each run of spinfiltration using a 100 µL tip.
3. Collect the concentrated QDs in a 1.5 mL tube, washing the filter twice with 2 drops of BBS.
4. Filter the QDs using a small filter, like a 4mm 0.22 µm Millex syringe filter. A 0.45 µm filter can be used if the solution has a large amount of aggregates.
5. Store the QDs in the fridge until needed.
6. Characterise the conjugation via DLS.
7. Dilute QDs to the desired concentration using the antibody diluent buffer before use.

Note 1: the reaction can be put in the fridge for 2 days instead of 18 hrs at RT.

Note 2: The molar ratio Ab:QD used here is 4:1.

### LFA protocol

### DAY 2: Spot the capture antibody

1. Spot 1 µL of a solution 1 mg/ml of the anti-ECM (MM01) antibody in PBS. Spot near the adsorption pad away from the antibody line.
2. Immediately after spotting, put the strips in an oven at 37°C for 2 hours.
3. Leave the strips to cool down, and close the strips in a tube with some desiccant.

### DAY 3: Virus detection in washing buffer

1. Filter the QDs to remove aggregates to reduce background noise.
2. Prepare solutions of ECM in QDs-anti-ECM-antibody (R030) with various concentration from 5 µM to 5 nM (in washing buffer: BBS buffer, Tween20 5%, BSA 5%).
3. Flow 35 µL of the ECM solution to the strip and wait until dry (10 minutes).
4. Wash with 50 µL of wash buffer, waiting 10 minutes to dry before each washing.
5. Check the fluorescence under UV light.

### EXPERIMENTAL

### Materials

- Linker stock solution = 0.1 mM in anhydrous DMSO, divided in single doses of 5 µL each (in 200 µL tubes). Store at -20°C.
- QDs solution = QD-azide; 3^{rd} generation, concentration = 0.9 µM. Store at 4°C.
- Anti-biotin antibody = polyclonal antibody raised in goat, product# B3640 from Sigma-Aldrich. Store at -20°C.
- BSA = bovine serum albumin for immunoassay (protease free, fatty acid free, essentially globulin free), product# A7030 from Sigma-Aldrich. Store at 4°C.
- Biotin-NHS = NHS-dPEG^{®}4-biotin, product# QBD10200 from Sigma-Aldrich, dissolved in anhydrous DMSO to a concentration of 1 mM and divided into single doses of 1 µL each in 200 µL tubes. Store at -20°C.
- Anti-Hendra polyclonal antibodies = raised in rabbit, about 20 mg/ml, divided into single doses of 1 µL each (in 200 µL tubes). Store at -20°C.
- Anti-Hendra monoclonal antibodies = human antibodies, 9.2 mg/ml, divided into single doses of 5 µL each (in 200 µL tubes). Store at -20°C.
- Human serum = product# S-2145 from Sigma-Aldrich. Store at -20°C.
- Hendra virus = concentrated and gamma-irradiated, stored at - 80°C.

### Buffers

Both Commercial and home-made buffers were used as listed below. Note: "borate buffered" = no NaCl. "Borate buffered saline" = with NaCl. "Normal saline" is solution isotonic to blood that contains 9 g/L NaCl in water (0.154 M). However, in the buffer recipes the NaCl is usually 8 g/L to get the same ionic strength.

### Commercial buffers

- PBS 1X = PBS 10 mM made up by dissolving capsules from ThermoScientific in MilliQ water, as required by manufacturer (https://www.thermofisher.com/order/catalog/product/189120 14).

### Home-made buffers

- Dulbecco PBS (1x) = 10 mM, pH∼7.4, 8 g/L NaCl

   Recipe: 1.15 g/L Na₂HPO₄ + 0.2 g/L KH₂PO₄ + 8 g/L NaCl + 0.2 g/L KCI.
- PBS-T = PBS + 0.05% v/v Tween20. Use 0.5 % v/v for a stronger washing.
   Note: density PBS 1X = 1.01 g/ml; density Tween20 = 1.1 g/ml → 0.05% v/v = 0.055 w/v = 0.055 w/w.
- Antibody dilution buffer = PBS-T + 5% w/v BSA and 30 mM glycine. For 1 ml of buffer, 50 mg BSA and 22.5 uL of glycine stock solution, brought to 1 ml with PBS-T is required.
- Glycine stock solution 1% w/w = 100 mg glycine in 1 g of PBS.
- MOPSr = MOPS 25 mM, pH 7.6, 0.8 g/L NaCl (r = reduced NaCl) Recipe:
- Tris buffer saline (aka Trizma) = 10 mM, pH∼8.3, 8 g/L NaCl.
   Recipe:
   1.2 g/L Tris + ∼8 mL of HCl 1M (check the pH) + 8 g/L NaCl. Bring to 1 L with milliQ.
      OR
   1.6 g/L Tris-HCI + 8 g/L NaCl (a solution of Tris-HCI should have pH∼8.3). Bring to 1 L with milliQ.
- Borate buffer saline = 10 mM, pH∼9.4, 8 g/L NaCl.
   Recipe: 0.62 g/L boric acid + 0.26 g/L NaOH + 8 g/L NaCl. Bring to 1 L with milliQ water. Check the pH.
   Note: use 5 mL of a freshly made 1 M solution of KOH if too difficult to weigh the KOH pellets.

### Equipment

- 200 µL polypropylene tubes.
- "Nanosep" spinfilters from Pall Corporation, 300 kDa Omega membrane (orange).
- "Amicon" spinfilters from Merk, 50 kDa membrane, 0.5 ml. This filter reduces the volume from 500 µL to 50 uL. This means that 3 rounds of purification reduce the concentration of unwanted molecules by 1,000-fold.
- 4 mm syringe filters "Millex", 0.22 µm, PVDF (http://www.merckmillipore.com/AU/en/product/Millex-Syringe-Filter-Durapore-PVDF-Non-sterile,MM NF-SLGVR04NL).
- Lateral flow strips with one control line. The control line is biotinylated mouse IgG1 from eBiosciences (CD4 PSD 038, Cat number: 13-4714-85, https://www.thermofisher.com/antibody/product/Mouse-IgG1-kappa-clone-P3-6-2-8-1-Isotype-Control/13-4714-85).

### METHODS

### Standard procedures

The aliquots of proteins were prepared inside a laminar flow cabinet using sterile tips and tubes to avoid bacterial contamination.

### Characterisation

A Biacore assay was used to check that the antibodies (polyclonal, biotinylated monoclonal) are active.

It will be understood to persons skilled in the art of the invention that many modifications may be made without departing from the spirit and scope of the invention.

It is to be understood that, if any prior art publication is referred to herein, such reference does not constitute an admission that the publication forms a part of the common general knowledge in the art, in Australia or any other country.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. A functionalised nanoparticle that is at least in part coated by a polymer, wherein the polymer comprises charged and uncharged groups at a ratio ranging from 4:1 to 1:4 and the functionalised nanoparticle is conjugatable or can be functionalised to conjugate with a biomolecule.

2. The functionalised nanoparticle according to claim 1, wherein the charged groups are negatively charged.

3. The functionalised nanoparticle according to claim 1 or claim 2, wherein at least one of the charged groups is a maleate group.

4. The functionalised nanoparticle according to any one of claims 1 to 3, wherein at least one of the uncharged groups is a sulfonate group.

5. The functionalised nanoparticle according to any one of claims 1 to 4, wherein the nanoparticle is a quantum dot.

6. The functionalised nanoparticle according to any one of claims 1 to 5, wherein the nanoparticle is bonded to a linker that is conjugatable with a biomolecule.

7. A method of producing a functionalised nanoparticle comprising:
providing a nanoparticle;
providing a polymer comprising charged and uncharged groups at a ratio ranging from 4:1 to 1:4; and
mixing the nanoparticle and the polymer to form the functionalised nanoparticle that is at least in part coated by the polymer and that is conjugatable or can be functionalised to conjugate to a biomolecule.

8. The method according to claim 7, including functionalising the polymer to be conjugatable with a biomolecule.

9. The method according to claim 7 or claim 8, including reacting the functionalised nanoparticle with a linker to form a nanoparticle-linker conjugate that is connectable to a biomolecule.

10. The method any one of claims 7 to 9, including reacting the polymer with a linker to form a polymer-linker conjugate that is connectable to a biomolecule.

11. A nanoparticle-biomolecule conjugate comprising a functionalised nanoparticle according to any one of claims 1 to 6 that is conjugated to a biomolecule, wherein the biomolecule can be bonded to a target analyte.

12. A method of producing a nanoparticle-biomolecule conjugate comprises:
providing a nanoparticle;
providing a polymer comprising charged and uncharged groups at a ratio ranging from 4:1 to 1:4;
mixing the nanoparticle and the polymer to form a functionalised nanoparticle which is at least in part coated by the polymer; and
reacting the functionalised nanoparticle with a biomolecule to form the nanoparticle-biomolecule conjugate, wherein the nanoparticle-biomolecule conjugate can be bonded to a target analyte.

13. The method according to claim 12, including reacting the functionalised nanoparticle with a linker to form a nanoparticle-linker conjugate.

14. The method according to claim 13, including reacting the nanoparticle-linker conjugate with a biomolecule to form the polymer-biomolecule conjugate.

15. The method according to any one of claims 12 to 14, including reacting a biomolecule with a linker to form a biomolecule-linker conjugate.
